(19) Europäisches Patentamt
European Patent Office
Office européen des brevets

(11) **EP 3 445 454 B1**

(12) **EUROPEAN PATENT SPECIFICATION**

(45) Date of publication and mention
of the grant of the patent:
**17.02.2021 Bulletin 2021/07**

(51) Int Cl.:
*A61Q 11/00* (2006.01)    *A46B 9/00* (2006.01)
*A46B 1/00* (2006.01)    *A61C 3/00* (2006.01)
*A61C 19/06* (2006.01)

(21) Application number: **17714693.3**

(22) Date of filing: **28.03.2017**

(86) International application number:
**PCT/EP2017/057327**

(87) International publication number:
**WO 2017/182239 (26.10.2017 Gazette 2017/43)**

(54) **ORAL CARE PRODUCT**

MUNDPFLEGEPRODUKT

PRODUIT D'HYGIÈNE BUCCALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.04.2016 EP 16165732**

(43) Date of publication of application:
**27.02.2019 Bulletin 2019/09**

(73) Proprietors:
• **Unilever PLC**
**London, Greater London EC4Y 0DY (GB)**
Designated Contracting States:
**CY GB IE MT**
• **Unilever N.V.**
**3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**

(72) Inventors:
• **BLASCO, Alessandro**
**26841 Casalpusterlengo (LO) (IT)**
• **LANDI, Giovanna**
**2000 Neuchâtel (CH)**
• **MUCCIO, Manuela**
**Wirral**
**Merseyside CH62 4ZD (GB)**

(74) Representative: **Tansley, Sally Elizabeth**
**Unilever PLC**
**Unilever Patent Group**
**Bronland 14**
**6708 WH Wageningen (NL)**

(56) References cited:
WO-A2-2013/050432    WO-A2-2013/050432
WO-A2-2013/050432    DE-A1-102011 011 323
DE-A1-102011 011 323    DE-A1-102011 011 323
FR-A1- 2 989 868    FR-A1- 2 989 868
FR-A1- 2 989 868    JP-A- 2001 000 457
JP-A- 2001 000 457    JP-A- 2001 000 457
US-A1- 2006 283 478    US-A1- 2006 283 478
US-A1- 2013 291 325    US-A1- 2013 291 325
US-A1- 2014 010 583    US-A1- 2014 010 583

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to an oral care product for treating dental hypersensitivity.

### Background and Prior Art

Documents that disclose oral care products are exemplified by JP 2001 000457 A, DE 10 2011 011323 A1, US 2014/010583 A1 and US 2013/291325 A1.

Dental hypersensitivity is a painful condition affecting up to 20% of the adult population. Hypersensitive teeth may be sensitive to cold, heat, air or sugary foods. Dental hypersensitivity is believed to be related to the general increase in exposed root surfaces of teeth as a result of periodontal disease, toothbrush abrasion, or cyclic loading fatigue of the thin enamel near the dento-enamel junction.

There are two categories of therapy for the treatment of tooth hypersensitivity based upon two modes of action. The first category, nerve-depolarising agents, are pharmaceutical agents such as potassium nitrate, which function by interfering with neural transduction of the pain stimulus.

The second category, known as occluding agents, function by physically blocking the exposed ends of the dentinal tubules, thereby reducing dentinal fluid movement and reducing the irritation associated with the shear stress described by the hydrodynamic theory.

An example of an occluding agent is found in US 5,270,031 which describes a tubule occluding desensitizer comprising a polyacrylic acid such as Carbopol® polymeric materials. Another tubule occluding composition is disclosed in US 5,374,417 which discloses a potassium salt of a synthetic anionic polymer, such as a polycarboxylate.

There is a continuing need for effective products aid application of composition to the teeth and which mitigate the effects of hypersensitivity.

### Description of the Invention

The present invention relates to an oral care product comprising a spatula and an oral care composition as defined in claim 1.

### Detailed Description

The oral care product of the invention comprises a spatula for applying an oral care product to the teeth. The shape of the spatula is such that product adheres to the spatula and yet can be easily applied to the teeth. The spatula comprises a wide, substantially flat application surface projecting from a shaft, the application surface having protruding from its surface at least one curved protrusion in which the length of the protrusion is greater than the height. Preferably, the edge of the application surface at the opposite end to the shaft is curved.

The spatula is formed from a semi-flexible material such that the application surface can move forwards and backwards freely. More preferably the spatula is made from an elastomeric material, most preferably the elastomeric material is a thermoelastic material.

Preferably, the application surface of the spatula extends perpendicularly to the centre line of the shaft.

Preferably, the width of the protrusions on the spatula are less than the height of the protrusion. It is also preferable if the length of the protrusion on the spatula is at least 5 times, more preferably 10 times the width of the protrusion. If the protrusion is in the form of a continuous ring the length is taken as the distance the circumference of the ring.

In one embodiment of the invention, the protrusions of the spatula are arranged in rows substantially perpendicular to a line positioned on the application surface close to the shaft. Preferably the line is perpendicular to the shaft. Preferably, the number of protrusions of the spatula is from 3 to 8.

Preferably a rim is present between the application surface and the shaft.

Preferably, the protrusions of the spatula are in the form of ribs.

In a second embodiment, the protrusion of the spatula is in the form of concentric rings.

Compositions of the invention are preferably in the form of a serum having a stable viscosity from 6000 to 100000 mPas, more preferably from 10 to 60000 mPas, most preferably from 15 to 40,000 mPas. Viscosity should be measured at 25°C, using a Brookfield viscometer with Helipath Stand and a T-bar, TB spindle, rotating at 5 rpm. The sample jar in which the measurement should be taken has a minimum diameter of 5 cm and a minimum height of 10 cm. The viscosity value should be recorded 60 seconds from the start of the measurement. In the context of the present invention, stable viscosity means that the viscosity does not significantly change on storage; this occurs usually after 3 weeks or greater after the manufacture of the composition.

Compositions according to the invention will usually contain an aqueous continuous phase. The amount of water generally ranges from 40 to 89% by weight based on the total weight of the composition, more preferably from 50 to 70 wt%.

Preferably, compositions according to the invention will further comprise humectants. The amount of humectant generally ranges from 10 to 50%, more preferably from 15 to 40% by weight based on the total weight of the composition. Preferred humectants that are present at the above levels include polyols, particularly preferred are sorbitol, glycerol and mixtures thereof.

It is preferred if the composition comprises a gum, more preferably gellan gum and/or xanthan gum. It is particularly preferred if the gum is Xanthan gum. It is preferred if the level of xanthan gum is from 0.05 to 1.2 wt% of the total composition, more preferably from 0.1 to 1.0 wt%.

The composition comprises ingredients that mitigate tooth sensitivity, a preferred ingredient for this effect is hydrated silica. Preferably the hydrated silica has a mean particle size lower than 15μm, more preferably lower than 10, most preferably from 1 to 6 μm. It is preferable if the hydrated silica described above is present at levels from 1 to 10 wt% of the total composition, more preferably form 3 to 8 wt% of the total composition.

Compositions of the invention may comprise a potassium salt selected from citrate, lactate or chloride, preferably the metal salt is a citrate salt. It is preferable if the level of potassium salt, in particular potassium citrate salt is from is from 1 to 10 wt% of the total composition, more preferably from 2 to 5 wt% of the total composition. Such salts help prevent dental sensitivity.

A further agent that mitigates tooth sensitivity is HAP (Calcium Hydroxyapatite). Preferably the level of HAP is from 0.5 to 10 wt% of the total composition, more preferably from 1 to 8 wt% of the total composition.

Compositions of the invention comprise a zinc salt, preferably zinc sulphate, zinc citrate or zinc chloride, more preferably zinc sulphate. Preferably the level of zinc salt is from 0.05 to 1.0 wt% of the total formulation more preferably from 0.1 to 0.5 wt%.

It is preferable if the level of ethanol in the total composition is less than 0.1 wt%.

Compositions of the invention may comprise a preservative. A preferred preservative is sodium benzoate, more preferably a system formed from ethylhexylglycerin, phenoxyethanol and benzyl alcohol. Preferably, the level of preservative in the case of the mixed system described, is from 0.5 to 2 wt% of the total composition. This level refers to the total level of preservative system.

It is preferable if the pH of the composition at 25°C is from 5,6 to 8,0, more preferably from 6,0 to 7,5.

The composition of the invention may comprise a deposition aid. The term "deposition aid" in the context of this invention generally means a material that aids deposition of agents that mitigate tooth sensitivity from the composition.

Use of the composition in the context of this invention typically involves application of the composition to the teeth by use of the applicator described above.

Suitable deposition aids for use in the invention will generally dissolve or disperse in water at a temperature of 25°C.

Preferred deposition aids for use in the invention are water soluble. The term "water-soluble" in this particular context generally means that the deposition aid has an aqueous solubility of at least 10g/L at 25°C, and more preferably at least 30g/L at 25°C (where the solubility is determined in unbuffered distilled water). It is particularly preferable that the deposition aid remains water soluble after drying, so that it can be re-dissolved. This prevents undesirable build up of the deposition aid on the teeth after repeated usage of the composition.

Suitable deposition aids for use in the invention include polymeric materials, preferably polymeric materials which are water soluble as defined above.

Polymeric materials for use as deposition aids in the invention may be naturally or synthetically-derived, and may be ionic or nonionic in nature.

Preferably such polymeric materials are high molecular weight. The term "high molecular weight" in this particular context generally means that the polymeric material has a molecular weight of at least 50,000, more preferably at least 500,000 g/mol. A suitable method to determine the molecular weight of such polymeric materials is gel permeation chromatography against a polyethylene glycol standard.

Specific examples of suitable classes of polymeric material for use as deposition aids in the invention include:

Water-soluble, high molecular weight linear homopolymers of ethylene oxide characterised by the general formula $H(OCH_2CH_2)_nOH$. These materials are generally termed polyethyleneoxides (or alternatively, polyoxyethylenes, or polyethylene glycols). In the general formula, n usually has an average value of at least 2000, preferably at least 50,000.

Water-soluble, high molecular weight cellulose ethers such as methylcellulose, hydroxyethylcellulose, hydroxypropylcellulose, hydroxypropyl methylcellulose, hydroxybutyl methylcellulose, hydroxyethyl ethylcellulose, sodium carboxymethylcellulose, and sodium carboxymethyl hydroxyethylcellulose.

A preferred class of polymeric material for use as deposition aids in the invention includes water-soluble, high molecular weight polymers having anionic side groups along the polymer main chain.

Specific examples of such materials include poly(carboxylic acid) polymers. Poly (carboxylic acid) polymers are typically polymers which include -COOH groups in their structure, or groups which are derived from -COOH groups such as salt, ester or anhydride groups.

For example, the poly(carboxylic acid) polymers may include:

$$-[C(R^1)(COOH)-]-$$

units in their structure, in which $R^1$ is selected from hydrogen, $C_{1-3}$ alkyl, $C_{1-3}$ alkoxy or $C_{1-3}$ hydroxyalkyl. Preferably $R^1$ is hydrogen.

A preferred type of poly (carboxylic acid) polymer includes adjacent:

$$-[C(R^1)(COOH)-]-$$

units in its structure (where $R^1$ is as defined above), for example polymers based on maleic acid, which typically include:

$$-\{-CH(COOH)-CH(COOH)-]-$$

units, and/or salts or esters of such units, or such units in anhydride form in which -COOH groups on adjacent carbon atoms are cyclised to form a ring system.

For example, the poly(carboxylic acid) polymers may comprise units with pairs of carboxylic acid groups on adjacent polymer chain carbon atoms, for example polymers comprising:

$$-[-C(R^1)(R^2)-C(R^3)(R^4)-C(R^5)(COOH)-C(R^6)(COOH)-]-$$

units in its structure (and/or salts or esters of such units, or such units in anhydride form in which - COOH groups on adjacent carbon atoms are cyclised to form a ring system); in which $R^1, R^2, R^3, R^4, R^5$ and $R^6$ are each independently selected from hydrogen, $C_{1-3}$ alkyl or $C_{1-3}$ alkoxy. Preferably $R^1$ and $R^2$ are hydrogen, $R^3$ is hydrogen and $R^4$ is methoxy and $R^5$ and $R^6$ are hydrogen. Such a poly(carboxylic acid) polymer may be described as the polymer based on a copolymer of methyl vinyl ether and maleic anhydride, and is commercially available for example under the trade name Gantrez®.

A particularly preferred example of such a polymer comprises:

$$-[-CH_2-CH(OCH_3)-CH(COOH)-CH(COOH)-]-$$

units in its structure, in which the -COOH groups are in free acid form. Such polymers may be linear or cross-linked. More preferably the polymer is linear. Polymers of this type are commercially available for example under the trade name Gantrez® S. Most preferably such a polymer has a molecular weight of at least 500,000 g/mol (e.g. Gantrez® S-96), ideally at least 1,000,000 g/mol (e.g. Gantrez® S-97).

Alternative polymers which may be used comprise the units as described above in anhydride form, i.e. in which the two adjacent -COOH groups are cyclised to form a ring system. Such polymers are commercially available under the tradename Gantrez® AN, e.g. Gantrez® AN-119, Gantrez® AN-903, Gantrez® AN-139 and Gantrez® AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the tradename Gantrez® MS, e.g. Gantrez® MS-955.

Other alternative polymers which may be used comprise the units as described above in partial ester form, for example in which some of the free -COOH groups are esterified with $C_{1-6}$ alkyl, e.g. ethyl or n-butyl. Such polymers are commercially available under the tradename Gantrez® ES, e.g. Gantrez® ES-225 or Gantrez® ES-425.

Mixtures of any of the above described materials may also be used.

The amount of deposition aid (as defined above) in compositions of the invention suitably ranges from 0.001 to 5.0%, preferably from 0.005 to 4.0wt%, more preferably from 0.01 to 2.0wt% by total weight deposition aid (as defined above) based on the total weight of the composition.

The composition may contain low levels of surfactant based on the total weight of the composition. If present, the surfactant is preferably present at levels of less than 3 wt% of the total composition, more preferably at levels from 0.2 to 2 wt%, most preferably at levels of less from 0.5 to 1.5 wt% of the total composition.

Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides.

Other suitable surfactants include nonionic surfactants, such as optionally polyethoxylated fatty acid sorbitan esters, ethoxylated fatty acids, esters of polyethylene glycol, ethers of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Steareth 30 an ether of polyethylene glycol is particularly preferred.

Compositions of the present invention may also contain further optional ingredients customary in the art such as fluoride ion sources, anticalculus agents, buffers, flavouring agents, sweetening agents, colouring agents, opacifying agents, further antisensitivity agents and antimicrobial agents.

Figure 1 plan view is an example of a comparative spatula.

Figure 2 is a plan view of a first embodiment of a spatula according to the invention.

Figure 3 is a plan view of a second embodiment of a spatula according to the invention.

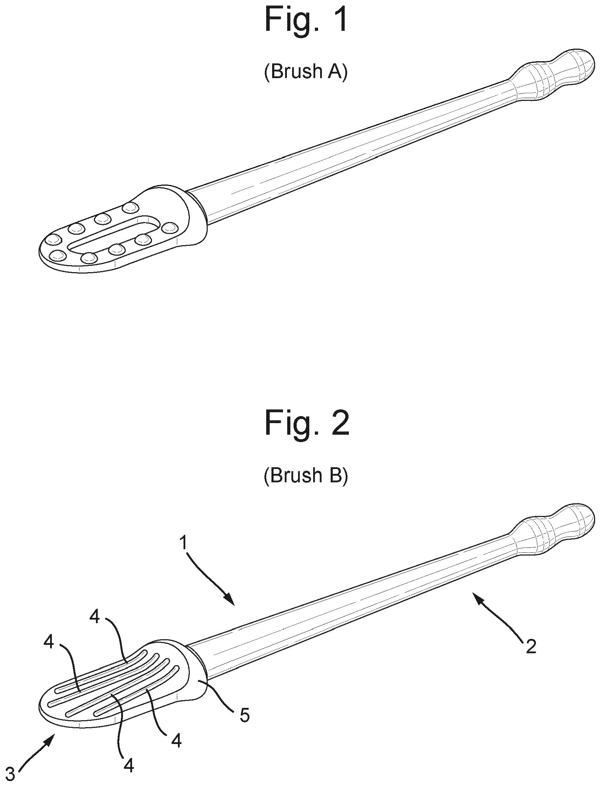

Figure 2 shows a spatula (1) having a shaft (2)having at one end a flat application surface (3). The flat application surface provided with a series of ribs (4). The ribs being positioned such that they are substantially perpendicular to a rim (5) between the application surface and the shaft.

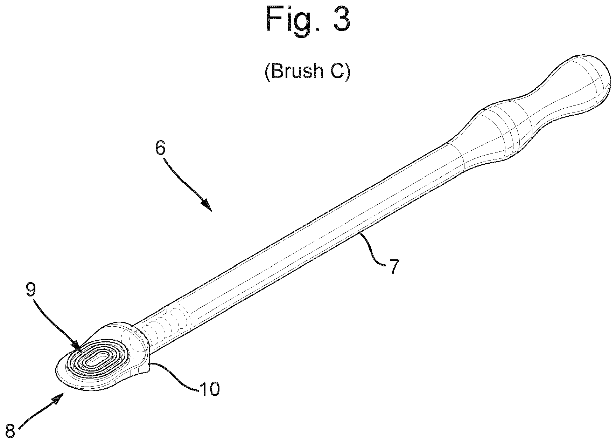

Figure 3 shows a spatula (6) having a shaft (7) having at one end a flat application surface (8). The flat application surface provided a coiled rib (9). There being a rim (10) between the application surface and the shaft.

The invention will now be illustrated by the following non-limiting Examples.

**Examples**

A composition was prepared according to Example 1

| Ingredient | Wt% |
|---|---|
| Water and minors | To 100 |
| Non-crystallisable sorbitol | 15.0 |
| Glycerine | 15.0 |
| Hydrated Silica | 5.0 |
| Potassium Citrate | 3.0 |
| Hydroxyapatite | 2.0 |
| Steareth-30 | 1.0 |
| Xanthan Gum | 0.75 |
| Methylvinylether and maleic acid copolymer | 0.5 |
| Zinc Sulphate | 0.20 |
| Sodium Monofluorophosphate | 0.17 |
| Gellan Gum | 0.05 |

Dentine discs from sound, caries-free human molars were polished to approx. 500 µm thickness. Hydraulic conductance equipment was used to measure the hydraulic conductance on the discs before and after application of Example 1 using one of spatulas of figure 1, 2 or 3.

Table 1 shows the average reduction in fluid flow rate, expressed as a percentage from the pre-treated baseline after treatment.

| Brush | Flow rate reduction / % | SD |
|---|---|---|
| Brush A | 31.73 | 9.58 |
| Brush B | 45.51 | 9.99 |
| Brush C | 49.07 | 10.95 |

A comparison of the fluid flow rate reduction for the three brush applications in this study shows that Brush C is directionally superior at reducing the fluid flow rate compared to B and is statistically superior compared to Brush A. The reduction in fluid flow rate followed the scheme:

Brush C = Brush B > Brush A.

## Claims

1. An oral care product comprising a spatula (6) and an oral care composition for use in mitigating tooth sensitivity,

   i) the spatula is formed from a semi-flexible material such that the application surface can move forwards and backwards freely and comprising a wide, substantially flat application surface (8) projecting from a shaft (7), the application surface having protruding from its surface at least one curved protrusion (9) in which the length of the protrusion is greater than the height;
   ii) the composition having a viscosity from 8,000 to 40,000 mPas at 25°C.

2. An oral care product according to claim 1, comprising a plurality of protrusions.

3. An oral care product according to claim 2, in which the protrusions comprise concentric rings.

4. An oral care product according to any preceding claim in which the number of protrusion(s) of the spatula is from 1 to 10.

5. An oral care product according to any preceding claim in which the number of protrusion(s) of the spatula is from 3 to 8.

6. An oral care product according to any preceding claim in which the width of the protrusion on the spatula is less than the height of the protrusion.

7. An oral care product according to any preceding claim in which the length of the protrusion on the spatula is at least 15 times the width of the protrusion.

8. An oral care product according to any preceding claim in which the spatula is made from an elastomeric material.

9. An oral care product according to any one of claims 1 to 3 in which the protrusion of the spatula is in the form of a coiled rib.

10. An oral care product according to any preceding claim in which the composition comprises a polymeric thickener.

11. An oral care product according to claim 10 in which the polymeric thickener is selected from the group consisting of xanthan gum, gellan gum and mixtures thereof.

12. An oral care product according to any preceding claim in which the composition comprises a deposition aid.

13. An oral care product according to claim 12 in which the deposition aid is polymer based on a copolymer of methyl vinyl ether and maleic anhydride.

**14.** An oral care product according to any preceding claim in which the composition further comprises an ingredient that mitigates tooth sensitivity.

**15.** An oral care product according to any preceding claim comprising hydrated silica.

**Patentansprüche**

**1.** Mundpflegeprodukt umfassend einen Spatel (6) und eine Mundpflegezusammensetzung zur Verwendung zur Linderung von Zahnempfindlichkeit, wobei

(i) der Spatel aus einem semiflexiblen Material gebildet ist, so dass die Anwendungsoberfläche sich ungehindert vorwärts und rückwärts bewegen kann, und eine breite, im wesentlichen flache Anwendungsoberfläche (8) umfasst, die von einem Stiel (7) hervorsteht, wobei die Anwendungsoberfläche mindestens einen von ihrer Oberfläche hervorstehenden gebogenen Vorsprung (9) aufweist, bei dem die Länge des Vorsprungs größer ist als die Höhe;
(ii) die Zusammensetzung eine Viskosität von 8000 bis 40000 mPas bei 25 °C aufweist.

**2.** Mundpflegeprodukt nach Anspruch 1, umfassend eine Mehrzahl von Vorsprüngen.

**3.** Mundpflegeprodukt nach Anspruch 2, bei dem die Vorsprünge konzentrische Ringe umfassen.

**4.** Mundpflegeprodukt nach irgendeinem vorhergehenden Anspruch, bei dem die Anzahl des oder der Vorsprünge des Spatels von 1 bis 10 ist.

**5.** Mundpflegeprodukt nach irgendeinem vorhergehenden Anspruch, bei dem die Anzahl des oder der Vorsprünge des Spatels von 3 bis 8 ist.

**6.** Mundpflegeprodukt nach irgendeinem vorhergehenden Anspruch, bei dem die Breite des Vorsprungs auf dem Spatel kleiner ist als die Höhe des Vorsprungs.

**7.** Mundpflegeprodukt nach irgendeinem vorhergehenden Anspruch, bei dem die Länge des Vorsprungs auf dem Spatel mindestens 15-mal die Breite des Vorsprungs ist.

**8.** Mundpflegeprodukt nach irgendeinem vorhergehenden Anspruch, bei dem der Spatel aus einem elastomeren Material ist.

**9.** Mundpflegeprodukt nach irgendeinem der Ansprüche 1 bis 3, bei dem der Vorsprung des Spatels in Form einer gewickelten Rippe ist.

**10.** Mundpflegeprodukt nach irgendeinem vorhergehenden Anspruch, bei dem die Zusammensetzung ein polymeres Verdickungsmittel umfasst.

**11.** Mundpflegeprodukt nach Anspruch 10, bei dem das polymere Verdickungsmittel ausgewählt ist aus der Gruppe bestehend aus Xanthangummi, Gellangummi und Mischungen davon.

**12.** Mundpflegeprodukt nach irgendeinem vorhergehenden Anspruch, bei dem die Zusammensetzung ein Abscheidungshilfsmittel umfasst.

**13.** Mundpflegeprodukt nach Anspruch 12, bei dem das Abscheidungshilfsmittel ein Polymer auf Basis von einem Copolymer von Methylvinylether und Maleinsäureanhydrid ist.

**14.** Mundpflegeprodukt nach irgendeinem vorhergehenden Anspruch, bei dem die Zusammensetzung ferner einen Bestandteil umfasst, der Zahnempfindlichkeit lindert.

**15.** Mundpflegeprodukt nach irgendeinem vorhergehenden Anspruch, umfassend Kieselsäure.

**Revendications**

1. Produit pour le soin oral comprenant une spatule (6) et une composition pour le soin oral pour une utilisation dans l'atténuation de la sensibilité dentaire,

   i) la spatule est formée d'un matériau semi-flexible tel que la surface d'application peut se déplacer vers l'avant et vers l'arrière librement et comprenant une large surface d'application, substantiellement plate (8) faisant saillie à partir d'un axe (7), la surface d'application présentant en saillie à partir de sa surface au moins une protubérance courbée (9) dans laquelle la longueur de la protubérance est supérieure à la hauteur ;
   ii) la composition ayant une viscosité de 8 000 à 40 000 mPa.s à 25°C.

2. Produit pour le soin oral selon la revendication 1, comprenant plusieurs protubérances.

3. Produit pour le soin oral selon la revendication 2, dans lequel les protubérances comprennent des anneaux concentriques.

4. Produit pour le soin oral selon l'une quelconque des revendications précédentes, dans lequel le nombre de protubérance(s) de la spatule est de 1 à 10.

5. Produit pour le soin oral selon l'une quelconque des revendications précédentes, dans lequel le nombre de protubérance(s) de la spatule est de 3 à 8.

6. Produit pour le soin oral selon l'une quelconque des revendications précédentes, dans lequel la largeur de la protubérance sur la spatule est inférieure à la hauteur de la protubérance.

7. Produit pour le soin oral selon l'une quelconque des revendications précédentes, dans lequel la longueur de la protubérance sur la spatule est au moins 15 fois la largeur de la protubérance.

8. Produit pour le soin oral selon l'une quelconque des revendications précédentes, dans lequel la spatule est constituée d'un matériau élastomère.

9. Produit pour le soin oral selon l'une quelconque des revendications 1 à 3, dans lequel la protubérance de la spatule est dans la forme d'une nervure enroulée.

10. Produit pour le soin oral selon l'une quelconque des revendications précédentes, dans lequel la composition comprend un épaississant polymère.

11. Produit pour le soin oral selon la revendication 10, dans lequel l'épaississant polymère est choisi dans le groupe consistant en gomme xanthane, gomme gellane et mélanges de celles-ci.

12. Produit pour le soin oral selon l'une quelconque des revendications précédentes, dans lequel la composition comprend un agent d'aide au dépôt.

13. Produit pour le soin oral selon la revendication 12, dans lequel l'agent d'aide au dépôt est un polymère à base d'un copolymère de méthylvinyléther et d'anhydride maléique.

14. Produit pour le soin oral selon l'une quelconque des revendications précédentes, dans lequel la composition comprend de plus un ingrédient qui atténue la sensibilité dentaire.

15. Produit pour le soin oral selon l'une quelconque des revendications précédentes comprenant de la silice hydratée.

# Fig. 1

## (Brush A)

# Fig. 2

## (Brush B)

# Fig. 3

(Brush C)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001000457 A
- DE 102011011323 A1
- US 2014010583 A1
- US 2013291325 A1
- US 5270031 A
- US 5374417 A